(19)

**Europäisches Patentamt**

**European Patent Office**

**Office européen des brevets**

(11) **EP 4 542 209 A1**

(12) **EUROPEAN PATENT APPLICATION**
published in accordance with Art. 153(4) EPC

(43) Date of publication:
23.04.2025 Bulletin 2025/17

(21) Application number: 23827171.2

(22) Date of filing: 19.06.2023

(51) International Patent Classification (IPC):
*G01N 23/201* (2018.01)

(52) Cooperative Patent Classification (CPC):
G01N 23/201

(86) International application number:
PCT/JP2023/022652

(87) International publication number:
WO 2023/248988 (28.12.2023 Gazette 2023/52)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 20.06.2022 JP 2022099081

(71) Applicant: Rigaku Corporation
Akishima-shi
Tokyo 196-8666 (JP)

(72) Inventors:
• SATO, Takashi
  Tokyo 196-8666 (JP)
• MATSUMOTO, Takashi
  Tokyo 196-8666 (JP)
• HASEGAWA, Tomokazu
  Tokyo 196-8666 (JP)

(74) Representative: Lambacher, Michael et al
V. Füner Ebbinghaus Finck Hano
Patentanwälte
Mariahilfplatz 3
81541 München (DE)

(54) **MORPHOLOGY ANALYSIS DEVICE, MORPHOLOGY ANALYSIS METHOD AND MORPHOLOGY ANALYSIS PROGRAM**

(57) A morphological analysis apparatus, a morphological analysis method and a morphological analysis program, which make it possible to recognize a morphology of a component of a molecule, are provided. A morphological analysis apparatus 200 for analyzing a morphology of a molecule in a solution, comprises a reference data storing section 234 for storing reference data which is molecular shape data of a subject molecule specified in advance, a subject data storing section 235 for storing subject data which is molecular shape data of the subject molecule to be analyzed, and a morphological estimation section 237 for estimating a morphology of a component of a molecule in the subject data by specifying a state of a physical quantity constituting a molecular shape of the subject data with respect to the reference data, wherein the reference data and the subject data each have a resolution of a molecular level.

FIG. 3

**EP 4 542 209 A1**

**Description**

RELATED ART

Field of the Invention

The present invention relates to a morphological analysis apparatus, a morphological analysis method, and a morphological analysis program for analyzing a morphology of a molecule in a solution.

Description of the Related Art

Biopharmaceuticals are proteins and contain macromolecules having a large molecular weight and complex structure as main components. Unlike small molecule drugs, biopharmaceuticals cannot produce chemically uniform proteins, and therefore conformation, heterogeneity of sugar chain structure and non-specific association greatly affect their effectiveness and safety. Therefore, in order to ensure reliability, post-translational modification, aggregation analysis and analysis of conformational change are required. Manufacturing of biopharmaceuticals requires large-scale capital investment and is very difficult to control in terms of quality (Non-Patent Document 1).

When biopharmaceuticals are administered, they require high dosages and several thousand times more of the drug substance than a single dose. In addition, it is necessary to evaluate the structural and qualitative properties of the final product, and it is also necessary to ensure comparability of the product including various parameters.

Even if biopharmaceuticals are produced using the same gene sequence, the conformation, sugar chain structure, or a drug substance that is not uniform in association is not the same pharmaceuticals. Therefore, in order to ensure the quality within a certain standard, the conditions such as culture, purification and concentration must be strictly controlled.

In particular, in the production of antibodies using CHO cell-lines, when the cells are cultured for a long period of time, the ability to produce antibodies is reduced and impurities are excreted. Therefore, it is necessary to keep culturing fresh cells at all times. In addition, it is difficult to control expression regulation, and antibodies of poor quality may be produced. Therefore, for comprehensive quality control, it is necessary not only to analyze conventional chemical properties and comprehensive physicochemical properties but also to confirm structural changes and fluctuations.

However, the analysis or analysis process of existing biopharmaceuticals takes a long time, and moreover, it is necessary to develop an analysis or analysis method according to the condition of the sample in advance prior to the analysis or analysis. In addition, there is a crucial restriction that a solution cannot be measured in the three-dimensional structure analysis technique as a very limited technique. Therefore, it is not possible to measure or analyze the characteristics of a drug substance in a target condition or state with respect to the characteristics related to the three-dimensional structure. In other words, it is not possible to know not only the conformation but also the distribution of movement, etc. of the molecule of drug substance at that time. Thus, morphological analysis of molecules effective for the development of drugs or for the control of manufacturing processes has not been carried out.

Conventionally, there is a method of observing a biological macromolecule by freezing a sample like cryoelectron microscopy, but the movement of the biological macromolecule does not appear because the sample is frozen. Further, a method of obtaining a three-dimensional distribution of electron density based on coordinates of atoms constituting a protein having a known structure by using a database is also known, but the movable structure cannot be recognized from the obtained three-dimensional distribution (Patent Document 1). Furthermore, it is obvious that, in any case, no observations are obtained under given conditions which are essential in the process of drug formulation development or quality control.

Non-Patent Documents

Non-patent document1: "Present state and problem of the biopharmaceutical industry", Junichi Matsuzaki, Journal of Biotechnology Society, 91 (9), 495-498, 2013

Patent Document

Patent Document 1: JP2005-250721A

As described above, there is a need for a technology for observing information related to three dimensional structure that can be applied to quality control, including drug formulation development and manufacturing processes of biopharmaceuticals. However, in the prior art, it is not possible to specify the electron density of the components which conformational changes and movement in the solution state of the biopharmaceutical molecule are reflected in. Therefore, the molecular morphology of the drug formulation solution component cannot be sufficiently analyzed. On the other hand, if there is a structure visualization technique that has strengths in visualization of the shape of molecules in a solution and analysis of fluctuations in molecules, it is possible to change the conformation and evaluate the movement in a solution in which biopharmaceutical molecules actually composed of proteins or the like exist.

The present invention has been made in view of such circumstances, and an object of the present invention is to provide a morphological analysis apparatus, a morphological analysis method, and a morphological analysis program capable of recognizing a morphology of a component of a molecule.

SUMMARY OF THE INVENTION

(1) In order to achieve the above object, the morphological analysis apparatus of the present invention is a morphological analysis apparatus for analyzing a morphology of molecules in a solution, and comprises a reference data storing section for storing reference data which is molecular shape data of a subject molecule specified in advance, a subject data storing section for storing subject data which is molecular shape data of the subject molecule to be analyzed, and a morphological estimation section for estimating a morphology of a component of a molecule in the subject data by specifying a state of a physical quantity constituting a molecular shape of the subject data with respect to the reference data, wherein the reference data and the subject data each have a resolution of a molecular level.

(2) Further, in the morphological analysis apparatus according to (1), the morphology of the component is a physical difference directly derived from a chemical difference between a component of a molecule identified by the subject data and a component of a molecule specified by the reference data.

(3) Further, in the morphological analysis apparatus according to (1), the morphology of the component is a difference derived from conformation and/or movement of a molecule specified by the subject data with respect to a molecule specified by the reference data.

(4) Further, in the morphological analysis apparatus according to (2) or (3), the molecule specified by the subject data is originally different from the molecule specified by the reference data.

(5) Further, in the morphological analysis apparatus according to (2) or (3), the molecule specified by the subject data is the same molecule at different point of time with respect to the molecule specified by the reference data.

(6) Further, in the morphological analysis apparatus according to any one of (1) to (5), the morphological estimation section specifies a state of a physical quantity constituting the molecular shape by how high or low is the physical quantity constituting the molecular shape or a shape or size of a spread represented as an aggregate of the physical quantity.

(7) Further, in the morphological analysis apparatus according to any one of (1) to (6), the morphological estimation section uses, as the reference data, data of a physical quantity constituting a molecular shape obtained by measuring a standard sample having low movement.

(8) Further, in the morphological analysis apparatus according to any one of (1) to (6), the morphological estimation section uses the accumulated analysis data as the reference data for the training data.

(9) Further, the morphological analysis apparatus according to any one of (1) to (8), further comprises an output information generating section for generating output information so as to be able to recognize a morphology of the subject data, wherein the output information generating section causes an output device to display the subject data and receives an input of designation information that designates a component to which the morphology is to be estimated with respect to the displayed subject data, and wherein the morphological estimation section estimates the morphology of the designated component based on the designation information.

(10) Further, in the morphological analysis apparatus according to any one of (1) to (9), further comprises an output information generating section for generating output information so as to recognize the morphology of the subject data, wherein the output information generating section causes an output device to display the subject data and further makes a particular mark indicating the estimated morphology to be displayed on the subject data.

(11) Further, in the morphological analysis apparatus according to any one of (1) to (10), the subject molecule is a Y-antibody molecule, and the component is a Fab or Fc.

(12) Further, in the morphological analysis apparatus according to (11), further comprises an output information generating section for generating output information so as to be able to recognize the morphology of the subject data, and a quality determining section for determining whether or not the subject molecule conforms to a quality standard based on the estimated morphology, wherein the output information generating section displays a non-conformance of the subject molecule with the quality standard when the subject molecule does not conform to the quality standard.

(13) Further, the morphological analysis method of the present invention is a morphological analysis method for analyzing a morphology of a molecule in a solution, and comprises the steps of reading out reference data which is molecular shape data of a subject molecule specified in advance, reading out subject data which is molecular shape data of the subject molecule to be analyzed, and estimating a morphology of a component of a molecule in the subject data by specifying a state of a physical quantity constituting a molecular shape of the subject data with respect to the reference data, wherein the reference data and the subject data each have a resolution of a molecular level.

(14) Further, the morphological analysis program of the present invention is a morphological analysis program for analyzing a morphology of a molecule in a solution, and causes a computer to execute the process of: reading out reference data which is molecular shape data of a subject molecule specified in advance, reading out subject data which is molecular shape data of the subject molecule to be analyzed, and estimating a morphology of a component of a molecule in the subject data by specifying a state of a physical quantity constituting a molecular shape of the subject data with respect to the reference data, wherein the reference data and the subject data each have a resolution of a molecular level.

Brief Description of the Drawings

FIG. 1 is a schematic diagram showing morphological analysis system of the present invention.
FIG. 2 is a perspective view showing an X-ray small angle scattering apparatus.
FIG. 3 is a block diagram showing details of a morphological analysis unit.
FIG. 4 is a flowchart showing a morphological analysis method.
FIGS. 5A and 5B are diagrams showing an electron-density projection and specified components of the subject data, respectively.
FIGS. 6A and 6B are diagrams showing marks (arrows), respectively.
FIGS. 7A to 7C are diagrams showing the electron-density projections and the marks (arrows) when the antibody molecule to be analyzed are viewed from the directions perpendicular to each other, respectively.
FIGS. 8A and 8B are diagrams showing marks (enclosing lines), respectively.
FIG. 9 is a schematic diagram showing a manufacturing process of a biopharmaceutical.
FIG. 10 is a block diagram showing details of a structure specifying unit.
FIG. 11 is a flowchart showing the structure specifying method.
FIG. 12 is a schematic diagram showing a structural model.
FIGS.13A and 13B are graphs of idealized distribution and $\chi^2$-Rg(ind) distribution that cannot be analyzed, respectively.
FIGS. 14A and 14B are diagrams showing electron density projections of a source antibody molecule and an antibody-drug conjugate, respectively.
FIGS. 15A and 15B are diagrams showing electron density projections of the antibody molecule at day 1 and at month 2 since production, respectively. FIG. 15B shows a mark indicating an opening degree of Fabs.
FIG. 16 is a schematic diagram showing structural models and points on Rg(ind)-$\chi^2$ graph.
FIG. 17 is a table showing calculation examples when left and right Fab lengths of the Y-shaped antibody molecule are weighted according to $\chi^2$.

DETAILED DESCRIPTION OF THE INVENTION

Next, embodiments of the present invention are described with reference to the drawings. To facilitate understanding of the description, the same reference numerals are assigned to the same components in the respective drawings, and duplicate descriptions are omitted.

Further, in the following, the present invention is described using the electron density as a representative of the physical quantities constituting the molecular shape, but the electron density distribution may be replaced with a physical quantity constituting a molecular shape such as a potential map, and the electron density data may be replaced with molecular shape data which is data of the physical quantities constituting the molecular shape. The physical quantity constituting the molecular shape refers to quantitative information according to a position that enables specifying a molecule-forming structure by interaction with radiation. The physical quantities constituting the molecular shape include, for example, an electron density map and a potential map that is a three-dimensional map representing a potential in the molecule. Molecular shape data may refer not only to a single structural model, but also to a plurality of structural models having a distribution, as described below.

[Principle]

When X-rays are irradiated to molecules that move freely in solution, scattered ray of a ring, rather than a spot, is generated. With respect to the measured X-ray scattering profile obtained by detecting such scattered ray, by selecting an electron density map obtained by recursive phase improvement, a structural model obtained by fitting an X-ray scattering profile calculated with an assumed coordinate structure model or a structural model having a high coincidence index of fitting among a plurality of structural models constituting the ensemble thereof, it is possible to accurately reproduce a structural model of a molecule in a solution having a structure with dynamic fluctuation.

When such a structure analysis method is used, the distribution of electron density of a molecule in a solution is specified. The components of the molecule may have an angle of each other in accordance with the reference or be open at the reference or more between both ends. Comparing these cases, it can be said that there is a difference in the conformation of each other. In some cases, only one component is fixed, and the other component is swingable in a certain direction. In such a case, the component may be said to have movement. The electron density of a motile component in the molecule specified in the structural model is locally reduced, and its distribution extends over the range of motion. In this way, by utilizing the distribution of the electron density, it is possible to estimate the morphology of the components of the molecule. The morphology of the components of the molecular to be estimated includes not only pure physical differences that are not due to chemical changes between the comparison subjects or physical differences that are indirectly caused by chemical differences, but also physical differences that are directly derived from chemical differences. The morphology of the component of the molecular are described below in detail.

[Morphological Analysis System]

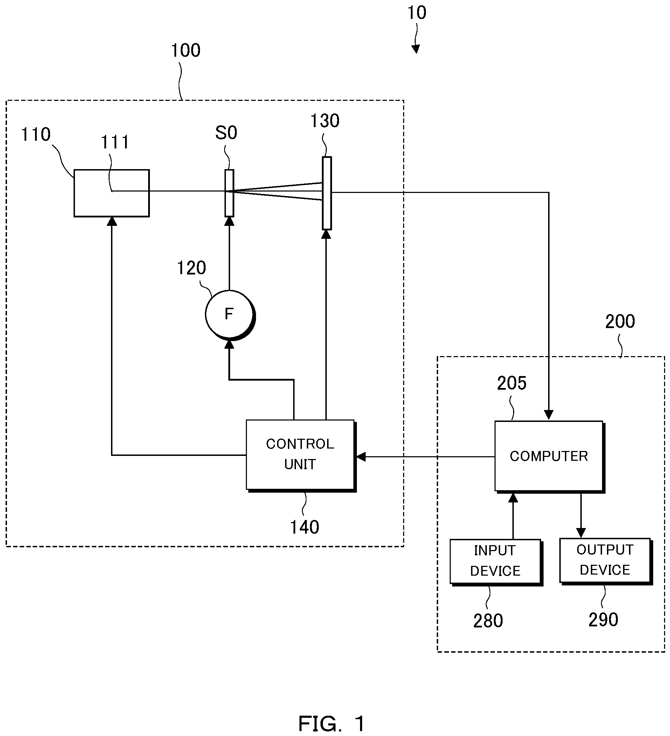

FIG. 1 is a schematic diagram showing morphological analysis system 10. The morphological analysis system 10 includes an X-ray small angle scattering apparatus 100 and a morphological analysis apparatus 200. The X-ray small angle scattering apparatus 100 measures the X-ray small angle scattering profile by irradiating the sample S0 with X-rays and detecting the small angle scattered X-rays. The sample S0 is suitable to be macromolecules in solution, in particular biomacromolecules. The sample S0 is preferably a molecule, molecular complex or structure for pharmaceutical use in solution. By using the X-ray solution scattering method by the morphological analysis system 10, it is possible to visualize a combination (ensemble) of structures of biomacromolecules that cannot be observed in a frozen state or a crystalline state.

The morphological analysis apparatus 200 comprises a computer 205, an input device 280, and an output device 290 and controls the operation of the X-ray small angle scattering apparatus 100 and acquires and processes measurement data from the X-ray small angle scattering apparatus 100.

The X-ray small angle scattering apparatus 100 comprises an X-ray generating section 110, a sample loading mechanism 120, a detector 130, and a control unit 140. The X-ray generating section 110 has an X-ray source 111 and irradiates the sample S0 with X-rays. The sample loading mechanism 120 delivers a solution with a macromolecule or a solvent without macromolecule as a sample to an X-ray irradiation position. The detector 130 detects X-rays scattered by the sample S0 and transmits the acquired measurement data to the computer 205.

The computer 205 is, for example, a PC, and comprises a processor that executes processes, a memory that stores programs and data, a hard disk, and the like. The computer 205 receives user input from an input device 280 such as a keyboard or a mouse. On the other hand, the computer 205 displays a plot, a visualized macromolecule image, an input screen, and the like on an output device 290 such as a display. The computer 205 may be a server device placed on a cloud. In addition, from the viewpoint of processing load, the function for controlling the operation of the X-ray small angle scattering apparatus 100 and the function for processing the measurement data may be separated, and the control may be executed by a PC placed at the site, and the data processing may be executed by the server device.

[Small Angle X-ray Scattering Apparatus]

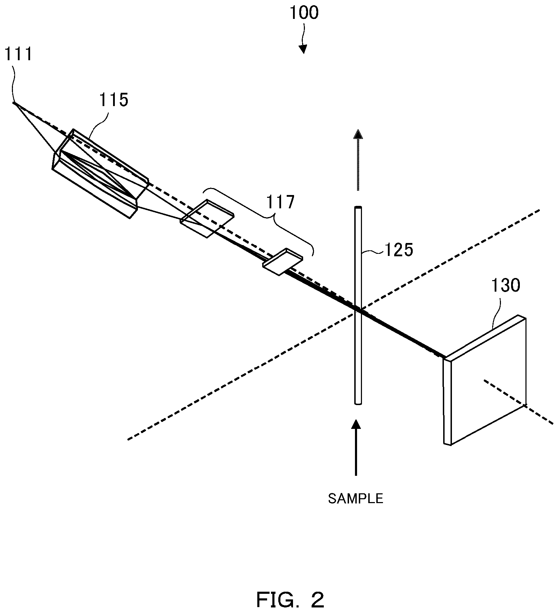

FIG. 2 is a perspective view showing an X-ray small angle scattering apparatus 100. The X-ray small angle scattering apparatus 100 comprises an X-ray source 111, an optical system 115, a Kratzky block 117, a sample holding tube 125 and a detector 130. The X-ray source 111 is a line radiation source or a point radiation source and emits a divergent beam. The optical system 115 is, for example, a KB parallel-type or series-type optical system. A pair of Kratzky blocks 117 interact with the X-rays by their respective edges to define one side and the other side of the X-ray beam. Thus, parasitic scattering can be removed from the irradiated X-rays. The sample holding tube 125 delivers and holds $5\mu l$ to $10\mu l$ of the solution sample. The detector 130 detects X-rays scattered by the solution sample.

[Morphological Analysis Apparatus]

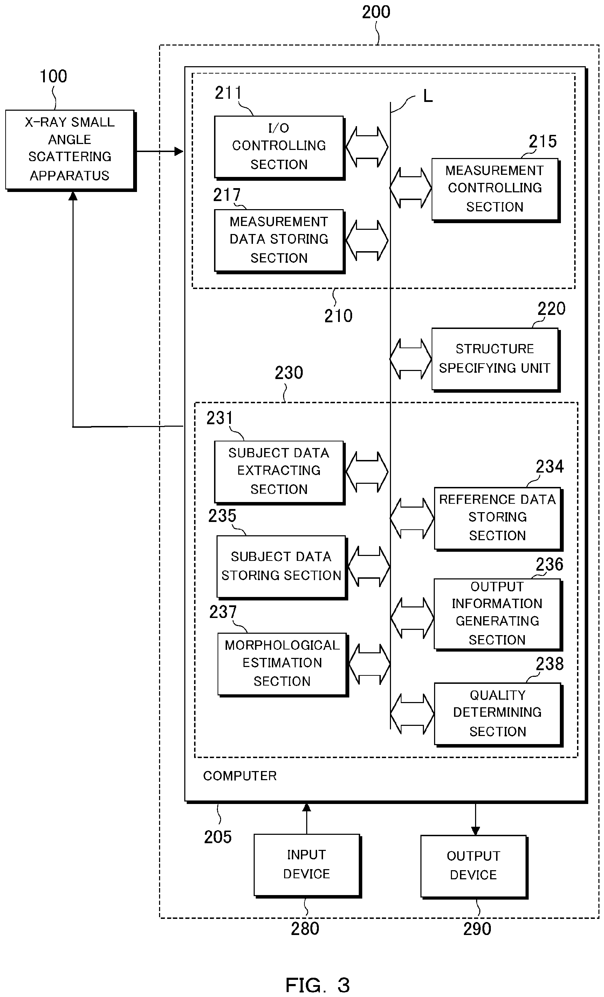

FIG. 3 is a block diagram showing details of a morphological analysis unit. The morphological analysis apparatus 200 analyzes the morphology of the macromolecule, of which the structure is specified, in the solution. The functions of the morphological analysis apparatus 200 are mainly achieved by the computer 205. The computer 205 comprises a basic functional unit 210, a structure specifying unit 220 and a morphological analysis section 230. The sections constituting each unit can transmit and receive information to and from each other by the control bus L.

(Basic Function Unit)

The basic function unit 210 executes basic functions such as input/output from/to a user and measurement control. The basic functional unit 210 comprises an input/output control section 211, a measurement control section 215, and a measurement data storing section 217. Note that each storing section in the morphological analysis apparatus 200 such as the measurement data storing section 217 is functionally divided into a plurality of sections and may physically correspond to the same memory or any of a plurality of memories.

The input/output control section 211 controls input/output from/to the outside. Specifically, an input from the input device 280 is received, and an output to the output device 290 is controlled. The I/O controlling section 211 can receive, for example, an input of a measurement condition or an input of a condition for generating a plurality of structural model. In addition, various plots can be output, and the structure of the specified macromolecule can be output.

The input/output control section 211 causes the output device 290 to display information generated for the output. The information generated for the output includes the electron density of the subject molecule obtained by the measurement. The input/output control section 211 receives input of designation information for designating a component to be analyzed in the morphology of the displayed subject data.

The measurement control section 215 controls the operation of the X-ray small angle scattering apparatus 100. The controls are for the sample delivery, the X-ray generation, and movement of the sample position and the detector. The control instruction is transmitted to the control unit 140 in the X-ray small angle scattering apparatus 100, whereby each part of the X-ray small angle scattering apparatus 100 is controlled.

The measurement data storing section 217 stores measurement data of the X-ray small angle scattering profile detected by the X-ray small angle scattering apparatus 100. The stored measurement data is used for generating of a structural model, calculation of an index and calculation of an actual measurement value of a molecular size of a macromolecule.

(Structure Specifying Unit)

The structure specifying unit 220 generates a plurality of structure models from the measured X-ray scattering profiles obtained by measuring the sample. Then, the degree of coincidence between the calculated X-ray scattering profile calculated from each of the plurality of structural models and the measured X-ray scattering profile is calculated, and a representative structural model is selected from the plurality of structural models according to the degree of coincidence. In this way, the structure specifying unit 220 accurately reproduces the structural model of the macromolecule in the solution having a structure with dynamical fluctuation. The structure specifying unit 220 are described below in detail.

(Morphological Analysis Unit)

The morphological analysis unit 230 comprises a subject data extracting section 231, a reference data storing section 234, a subject data storing section 235, an output information generating section 236, a morphological estimation section 237 and a quality determining section 238. The morphological analysis unit 230 analyzes the morphology of the molecules in the solution from the electron density of the subject data obtained by the structure specifying unit 220.

The subject data extracting section 231 extracts subject data to analyze the morphology of the component from the structural models specified by the structure specifying unit 220. The subject data is generated by the structure specifying unit 220 selecting a representative structural model from a plurality of structural models. The electron density data that can be the subject data may be one representative structural model or may be a plurality of representative structural models having a distribution. The distribution may be a plurality of structural models weighted according to $\chi^2$ in an objective evaluation without arbitrariness or may be a plurality of equivalent structural models in a predetermined range from $\chi^2=1$.

For example, weights can be defined such that the closer $\chi^2$ is to 1, the greater the weights, and the respective parameters can be represented by weighted averages. In the weighting, the following formula (1) or (2) can be used for a weighting function as a function of monotonically decreasing with respect to the absolute difference between $\chi^2$ and 1. In any case, a is a real number (usually 0), b and c are positive real numbers, and n is a logarithmic base.

$$w = 1 / |1 + a + \log_n(\chi^2)|^b \quad \cdots (1)$$

$$w = 1 / (1 + a + |\chi^2 - 1|^b)^c \quad \cdots (2)$$

In the above-described embodiment, the reference value of $\chi^2$ is 1, but the reference value may deviate from 1 when the molecule to be analyzed can take two states and a multiple distribution occurs. In the case, the weighting is defined so that the closer the $\chi^2$ is to the reference value, the larger the weighting is. For example, PCT characteristics may be analyzed and an offset(a) may be required when the two components coexist.

The reference data storing section 234 stores the reference data. The reference data is electron density data of a previously specified subject molecule. The reference data is registered, for example, as the electron density data of a known subject molecule. The reference data is preferably analysis data previously analyzed and accumulated by the morphological analysis apparatus 200 but may be data composed of known information. "Previously" means prior to the morphological analysis currently being performed. The electron density data that can be the reference data may be one representative structural model as in the case of the subject data or may be a plurality of representative structural models having a distribution.

The subject data storing section 235 stores subject data representing the electron density of the subject molecule. The subject data is electron density data of the subject molecule. Both the reference data and the subject data have a molecular level resolution. The "molecular level resolution" is preferably a resolution of 3 Å or more and 10 Å or less with respect to, for example, a size of 140 Å or more and 350 Å or less of a component of the subject molecule for which morphology is to be analyzed. Note that the subject data is data obtained by measurement, but the reference data does not necessarily have to be measured. The reference data may be, for example, data calculated from a known crystal structure.

The output information generating section 236 generates output information so that the morphology of the subject data can be recognized. The output information generating section 236 preferably generates output information based on the estimated morphology of the subject molecule. The output information generating section 236 converts, for example, an electron density distribution projected in a predetermined direction into a shade of a single color or a shade of a warm-to-cold color according to a position. For example, a position having a large electron density may be represented by a dark single color or a dark warm color. In addition, a position having a small electron density can be represented by a light single color or a light warm color. It is also possible to express the distribution of the electron density as a two-color gradient, such as a blue-to-red gradient. As a result, it is possible to express a difference derived from the conformation and/or the movement with respect to a component of the subject molecule.

Note that the output information generating section 236 may generate output information for visually displaying the difference in the subject data with respect to the reference data without being based on the estimated morphology of the subject molecule. For example, the output information may be generated by superimposing the subject data on the reference data.

The output information generating section 236 extracts a state and condition associated with the subject molecule and displays these state and condition together with the electron density distribution of the subject molecule. For example, the state and condition of the antibody molecule include the presence or absence of a sugar chain, a time dependent change, a culture condition, a nutrient condition, an extraction method and a transportation method.

The output information generating section 236 may display the mark together with the electron density of the subject molecule as a result of analyzing the morphology of the component. The mark is preferably superimposed on the display of the electron density of the component. Thus, the parameters of the morphology of the molecular can be visually clearly displayed. For example, it is possible to visualize the conformational features in an opening degree of a Y-shaped element or to display the direction and distance of the movement by a line segment with arrows on both ends (a mark of an arrow). For example, it is also possible to display the propagation of the movement of the subject molecule with an arrow.

In addition, a balloon-shaped enclosing line (an enclosing line mark) can be displayed as a mark at a void portion or a portion having a low density. In this way, it is effective to use a mark capable of displaying the size of the space for the void or the like. The mark of the enclosing line is a sign indicating the structure of the molecule and represents the physical difference directly derived from the chemical difference of the component in which the void or the like is induced. In addition, in the case of a time dependent change, a portion where the movement is increased can be displayed by a mark.

Note that, in a case where the subject molecule whose morphology has been analyzed does not conform to the quality standard, the nonconformity of the subject molecule to the quality standard may be displayed. When the quality standard is not conformed, there is a case where the parameter representing the morphology of the component of the molecule does not fall within a predetermined range of values.

The morphological estimating section 237 reads out the reference data and the subject data and estimates the morphology of the components of the molecule by specifying the state of the electron density of the subject data with respect to the reference data. Thus, a difference derived from conformation and/or movement can be recognized as the morphology of the components of the molecular. As a result, for example, in a manufacturing process of a biopharmaceutical, a difference derived from conformation and/or movement of the evaluated molecular structure can be used for quality control.

The morphological estimation section 237 specifies the state of the electron density distribution by the shape or size of the electron density. Thus, the morphology of the components of the molecule can be recognized, and as a result, the morphology of the subject molecule can be estimated. Examples of the size include a cross-sectional area and a distance.

The morphological estimation section 237 can specify, for example, a Fab and Fc of the antibody molecule as components of the subject molecule. For example, each component can be specified by determining a center position of a subject molecule and determining an edge based on the center position. The component can be regarded as an ellipsoid, and the length of the minor axis including its center can be calculated as the size of the component. It is also possible to specify the opening degree by specifying the central axis of each component and determining the angle formed by each other. The information in such a morphology may be calculated as a difference or a ratio of the subject data with respect to the reference data.

Conformation is the spatial arrangement of each domain in a molecule. The spatial arrangement is related to the position information of the electron density and includes the relative positions and angles of the components. A conformational change refers to a change in the spatial arrangement of a domain at one measurement time to a spatial arrangement of a domain at another measurement time. Movement is associated with high and low electron density and is the movement of mobile components within a molecule. The movement refers to a state of movement without converging to a predetermined arrangement within a certain measurement time range. The movement includes the speed, distance, range, and direction of movement in motion.

The morphological estimation section 237 may evaluate the structural change of the subject molecule, specify the degree of time dependent change, evaluate the flexibility or specify a flexible portion based on a difference derived from the evaluated conformation and/or movement of the subject molecule. For example, differences in the opening degree and flexibility of respective Fabs of the antibody molecule can be specified. Note that the morphological estimation section 237 may evaluate the abnormality of the entire molecule as a damage to the structure.

The morphological estimation section 237 can use, as reference data, data of electron density obtained by measuring a standard sample having low movement. Thus, the morphology of the components of the molecule can be evaluated by using the measurement result of the standard sample even if there is no known electron density data.

The morphological estimation section 237 may have an AI function represented by a machine-learning model. When the morphological estimation section 237 has an AI function, the accumulated analysis data can be used as the reference data for the training data. Thus, even when there is no reference sample or clear reference data, it is possible to analyze and evaluate the morphology of the components of the subject molecule in the subject data by AI using the accumulated analysis data as training data.

For example, a machine-learning model including a neural network including an input layer, an intermediate layer and an output layer can be used. When the electron density distribution of the subject molecule and its morphology information or quality information are known, they get associated with each other and used in the training data and are made to be learned to the machine-learning model. That is, the weight of each neuron in the intermediate layer is tuned by repeatedly learning so that the electron density distribution of the subject molecule is input, and correct morphology information or quality information is output at the output layer with high probability. In this case, a convolutional neural network may be used to roughly recognize the subject. Thus, if the electronic density distribution of the subject data is input to the machine-learning model even without using a parameter or the like in particular, the morphology of the subject molecule can be estimated, or the quality can be determined.

It is preferable that the morphological estimation section 237 evaluates a difference derived from the conformation and/or the movement designated based on the designation information. Thus, for example, it is possible to obtain an evaluation result for a component of a subject molecule, for which a difference derived from conformation and/or movement is desired to be evaluated, specified by range designation by a mouse.

The morphological estimation section 237 may compare the molecular structure or movement evaluated at a certain time t1 with the molecular structure or movement at a certain time t2 after a period of time elapses (t1<t2) for the same sample. By clarifying the difference derived from the conformation and/or the movement due to the time dependent change obtained in this way, the user can confirm the influence of time.

The morphological estimation section 237 can also evaluate the stability of the subject molecule. For example, the morphological estimation section 237 can determine the presence or absence of a component of the subject molecule. If there is not a component, it is preferable to also estimate the position and size of the void. For example, the stability of Fc achieved by the glycosylation of the antibody molecule can be evaluated. It is also possible to determine the deficiency of the sugar chain originally bound to Fc of the antibody molecule. The morphological estimation section 237 can also evaluate the non-equivalence between the components. For example, a non-equivalence evaluation for a Fab of the antibody molecule can be performed. In this way, when there is a region in which movement increases due to the state of the antibody molecule being different from the reference state, the region is specified and rendered displayable.

The quality determination section 238 determines whether or not the subject molecule conforms to the quality standard based on the estimated morphology of the subject molecule. For example, it is determined whether or not the parameter indicated by the estimated morphology falls within a predetermined value range with respect to the Y-shaped antibody molecule. The parameter indicated by the morphology includes a numerical value indicating the size, angle, or movement of the component. For example, whether or not the opening degree of Fabs for estimating the difference in conformation is within a predetermined range corresponds to the parameter. The predetermined value is a standard threshold based on the reference data or a threshold including a confidence limit. For example, in the production process of a Y-shaped antibody molecule, if an antibody has an angle open by 140° or more as a predetermined angle formed by the left and right Fabs, it is considered to be deteriorated.

When the parameter does not fall within the predetermined value range, the quality determining section 238 can specify the product as a defective product. For example, the subject molecule is a Y-antibody molecule, and the component is a Fab or Fc. By measuring and analyzing in such a setting, the quality of the product can be determined in the manufacturing process of the Y-shaped antibody molecule. It should be noted that the conformity to the quality may be determined by inputting the information corresponding to the morphology of the subject molecule and the information of the quality standard into the learning model by AI without using the predetermined value.

[Morphological Analysis Method]

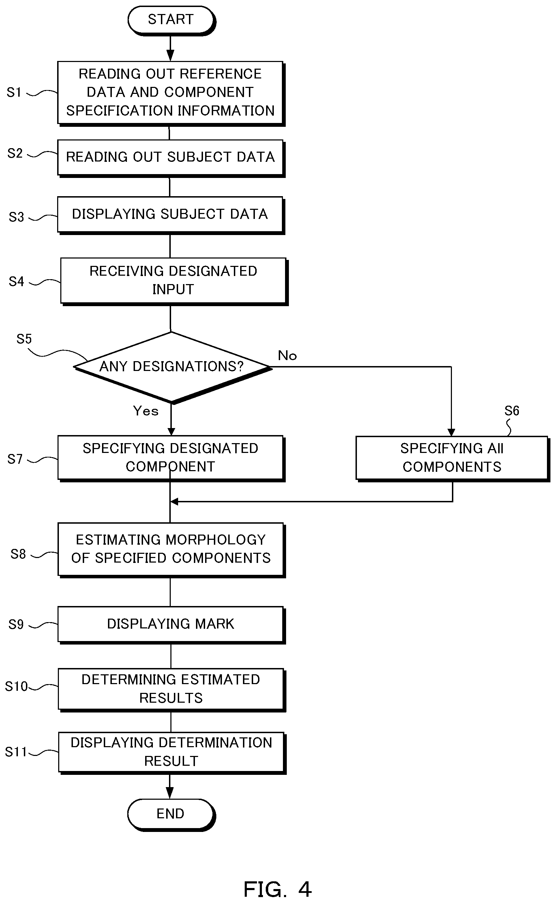

The operation of the morphological analysis apparatus 200 configured as described above is described below. Fig. 4 is a flowchart showing the morphological analysis method of movement. First, the morphological analysis apparatus 200 reads out the reference data and the component specification information (step S1).

The reference data is, for example, known electron density data of an antibody molecule whose morphology is to be analyzed. Morphological analysis includes evaluation of difference derived from conformation and/or movement. The component specification information is information for specifying a portion corresponding to each component from the electron density data of the subject molecule. The read information may further include information specifying a component to be analyzed in advance.

Next, the subject data is read out (step S2), and the subject data is displayed (step S3). The subject data is displayed as a projection image in a predetermined direction. Then, the morphological analysis apparatus 200 receives an input for designating a component to be analyzed for a morphology with respect to the subject molecule (step S4). For example, the components of the antibody-molecule include a Fab. When the designation is unnecessary, the designation may be skipped.

The morphological analysis apparatus 200 determines whether or not there is any information for specifying a component (step S5), and when there is no specification of a component, specifies all the components (step S6). On the other hand, if there is information specifying a component, the specified component is specified (step S7). For example, a Fc, left Fab and right Fab of the antibody molecule are assigned as components. The specification of the components is described below in detail.

Then, the morphological analysis apparatus 200 estimates the morphology using the states of the electron densities of the specified components (step S8). For example, an electron density distribution representing a Fab of the antibody-molecule is sliced in a plane including the central axis of the Fab, and the longitudinal width and the lateral width on the cross-section are detected. The longitudinal direction is a central axis direction, and the lateral direction is a direction perpendicular to the longitudinal direction. In addition, the cross-sectional area of the component is estimated from the longitudinal and lateral widths of the cross-section. The longitudinal and lateral widths can be fitted to the major and minor axes to calculate the area of the ellipse.

The morphological analysis apparatus 200 may display a mark indicating the morphology according to the estimated morphology (step S9). The marks representing movement include, for example, a mark of an arrow. The marks indicating the change in the conformation include, for example, a mark of an enclosing line. The various marks are described below in detail.

The morphological analysis apparatus 200 determines a morphological estimation result (step S10). For example, if the cross-sectional area of the Fab is larger than the reference, it is determined that the movement is high, and if the cross-sectional area is small, it is determined that the movement is low. Further, for example, it may be confirmed that the sample is not a defective product based on the determination. Then, the determination is displayed (step S11), and the series of operations is ended.

In the evaluation of movement, the lateral width of the cross-section may be compared between the left Fab and the right Fab, and the one with the longer lateral width may be evaluated as a Fab with high movement, and the one with the shorter lateral width may be evaluated as a Fab with lower movement. It may then be determined whether there is a difference in movement in both of the Fabs.

In addition, for example, the orientation of the Fabs forming the Y-shape in the Y-shaped antibody-molecule (rising degree) can be evaluated. In the case, it may be evaluated as the angle of the central axis of the Fab with respect to the central axis of the Fc, or it may be evaluated as the angle formed between the Fabs. When the angle of the Fab with respect to the Fc is a predetermined value or less, or when the angle formed between Fabs is a predetermined value or greater, it is determined that the Fab is lowered from the original position and the antibody-molecule is close to the T-shaped, and the sample can be regarded as a defective product. In this way, the opening degree of the Y-shape of the antibody molecule can be treated as an index for evaluating the freshness of the sample.

[Specification of Components]

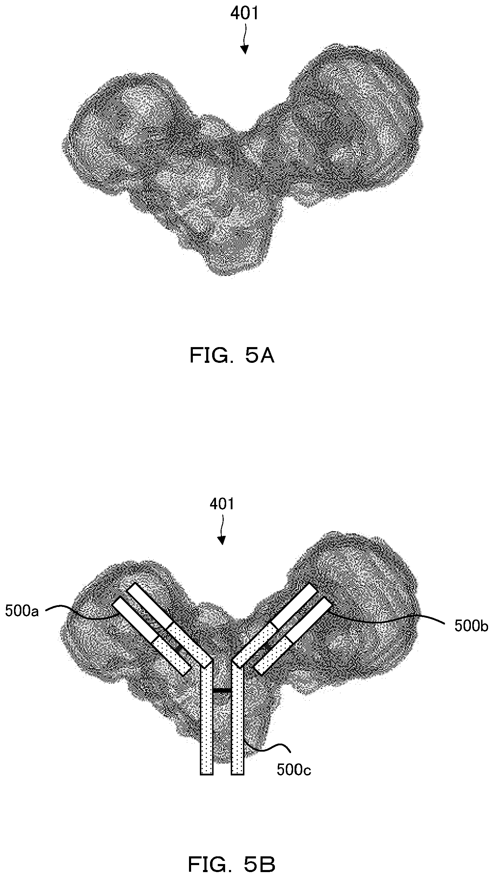

FIGS. 5A and 5B are diagrams showing an electron-density projection image and specified components of the subject data, respectively. FIG. 5A is a projection image showing an electron-density distribution of the subject data of a human serum antibody molecule 401. The projection image is an image obtained by projecting an electron density of a human serum antibody molecule in a certain direction. The human serum antibody molecule 401 represented in the projection image retains Y-shaped.

With respect to the electron-density distribution, the Fab and Fc regions are detected as components by a Y-search. Fig. 5B shows the detection results of the respective regions. As shown in FIG. 5B, the regions of the Fabs and Fc arranged vertically above and below in the solution, respectively, have been detected, and marks 500a, 500b and 500c (line segments) indicating respective regions of the left Fab, right Fab and Fc are displayed. The left Fab and the right Fab can be specified because the projection direction is determined.

[Morphology of Components]

(Observation of Physical Difference Directly from Chemical Difference)

By comparing the subject data with the reference data, the morphologies of the components of the subject data can be estimated. The morphologies of the components may be expressed as a physical difference derived from a chemical difference of a component of a molecule specified by the subject data with respect to a molecule specified by the reference data. The chemical differences include short-cut, defect, extension or any substance addition in a portion of the molecule specified in the subject data with respect to the reference molecule. When these chemical changes are prominent, the short-cut or the defect appears as a lack of the electron density of the partial, and the extension or the addition of any substance appears as an extra electron density. For example, when developing new designs of Bispecific antibody, etc. or culturing conditions, it is possible to determine the success/failure by quickly knowing the state of addition of sugar chains, modification by unintended chemical substances, etc., and to optimize the process. Physical differences directly derived from such chemical differences occur mainly in comparisons of different subjects.

(Observation of Physical Differences Regardless of Chemical Differences)

The morphology of the component may be a difference derived from the conformation and/or movement of the molecular specified in the subject data of with respect to the molecule specified in the reference data. In this case, even if a chemical configuration difference such as a defect is very small and is not observed as a physical difference such as a direct electron density defect, it may be appeared by the conformation and/or movement as a result of an indirect effect. In this case, the molecule specified by the subject data may be originally different from the molecule specified by the reference data in some cases or may be the same but just at different comparison timing as the molecule specified by the reference data in other cases.

The differences derived from the conformation and/or movement includes a difference in conformation, a difference in distribution of conformation, a difference in movement, or a difference in distribution of movement.

(When Compared Subject is Originally Different)

If the compared subjects are originally different, it is useful to see differences from the conformation and/or movement. For example, this is a case where a molecule specified by the reference data is used as a reference, and a molecule specified by the subject data is observed. This includes a case of designing the antibody drug function itself for the same antibody and a case of analyzing a molecule that provide a different function even though the antibody drug same as the reference has been produced.

(When Compared Subjects are Same but at Different Time Points)

Even when the compared subjects are the same but at different time points, it is effective to see a difference derived from the conformation and/or movement. For example, a time dependent change of a single molecule can be observed. In this case, a change and distribution of the conformation and/or movement can be observed. It is effective for drug formulation development of antibody drug and quality control of manufactured antibody drug.

[Display of Mark]

The morphology estimated using the state of the electron density of the components can be represented using marks. In the embodiment shown in FIG. 5B, the components of the left Fab, the right Fab and the Fc are indicated by marks 500a, 500b and 500c (line segments), respectively. In this way, the mark can indicate the conformation of the subject molecule. The marks 500a, 500b and 500c can also be represented by double lines along the molecular chain.

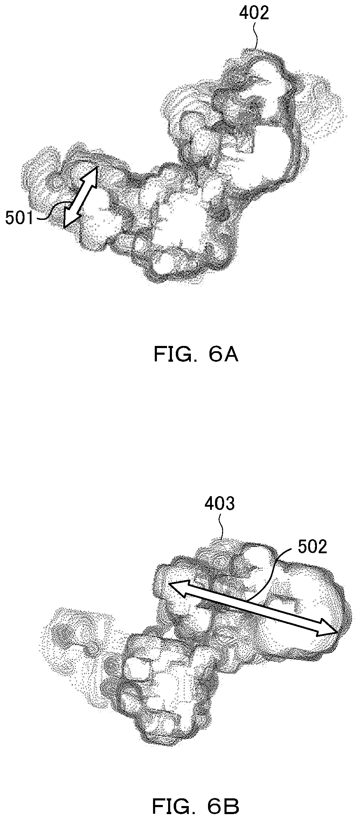

The mark may also indicate the movement of the subject molecule. FIGS. 6A and 6B are diagrams showing marks (arrows) 501 and 502, respectively. In the embodiment shown in FIG.6A, a line segment with arrows at both ends indicating the movement of the left Fab of the human serum antibody molecule 402 is displayed on the projected image of the electron-density of the subject data obtained with the human serum antibody molecule 402. The length of the line segment corresponding to the lateral width in the cross-section including the central axis of the Fab represents the magnitude of the movement, and the arrows at both ends can represent the directionality of the motion. This mark 501 visualizes that the left Fab is narrow in lateral width and spatially non-extensive and clearly indicates its low movement.

In the embodiment shown in FIG. 6B, a line segment with arrows at both ends indicating the movement of the right Fab of the human serum antibody molecule 403 is displayed on the projected image of the electron-density of the subject data obtained with the human serum antibody molecule 403. This mark 502 allows the right Fab to be significantly wider than the left Fab, visualizes the spatial extent and clearly displays its high movement.

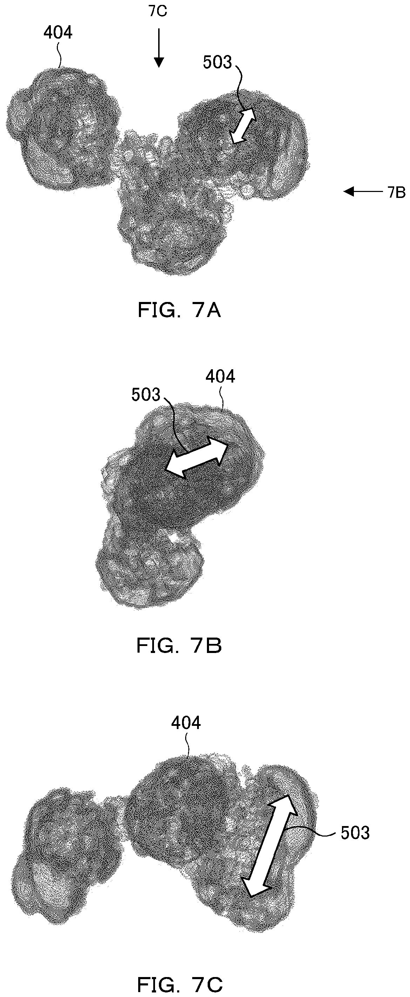

The mark indicating the evaluated movement as described above is a three-dimensional vector amount, and the shape on the screen differs depending on the projection direction of the antibody molecule to be analyzed. FIGS. 7A to 7C are diagrams showing the electron-density projections and the marks (arrows) when the antibody molecule 404 to be analyzed are viewed from the directions perpendicular to each other, respectively. FIG. 7B and 7C are views showing the antibody-molecule 404 and mark 503 as viewed from the direction 7B and 7C shown in the FIG. 7A, respectively (right side view and plan view when FIG. 7A is taken as a front view). Thus, it can be seen that, in particular, the region of the Fab is wider in the projected image of FIG. 7C and is moving in a direction perpendicular to the plane formed by the Y-shape.

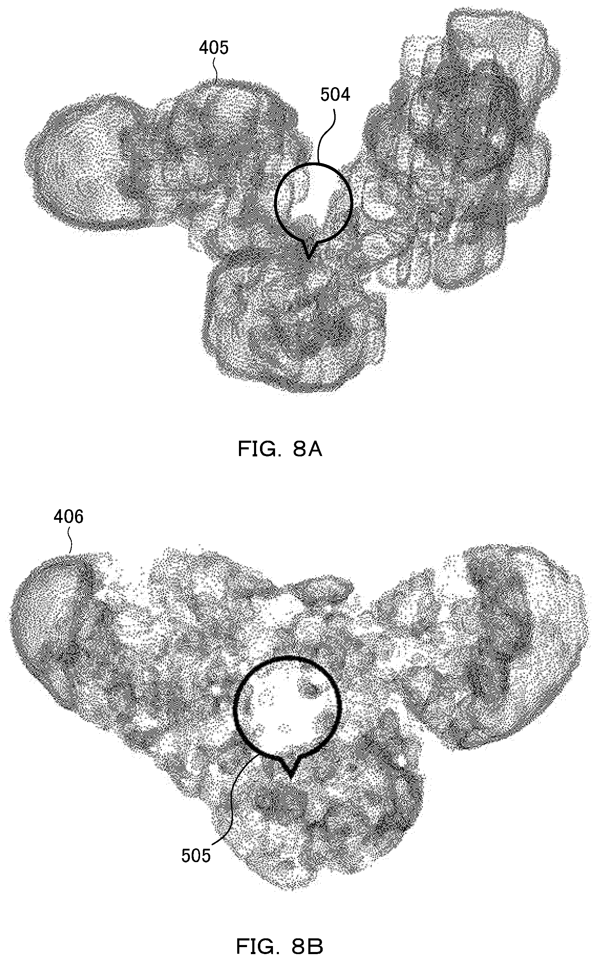

Marks can also be used to indicate physical differences directly derived from chemical differences in molecular components. Physical differences directly derived from chemical differences in molecular components include cleavage or defects in the components. FIGS. 8A and 8B are diagrams showing marks (enclosing lines) 504 and 505, respectively. In the case shown in FIG. 8A, the sugar chain attached to the Fc in the antibody-molecule 405 is cleaved, so that the sugar chain in the central of the Fc disappears and the Fc is cleaved. If there is a defect or the like in the structural of the subject data with respect to the structure of the reference data, the region can be indicated by an enclosing line. In the embodiment shown in FIG. 8A, the position and the size of the cleavage part are indicated by displaying a mark 504 formed by a balloon-shaped enclosing line on the cleavage part of the Fc.

In addition, in the shown embodiment, it can be seen that a void is formed in the center of the Fc in the antibody molecule 406. This void implies that there may be no sugar chains inherently attached to the Fc. The position and size of the void can be indicated by displaying a mark 505 formed by a balloon-shaped enclosing line on the void. Note that the mark is not limited to an arrow or an enclosing line, and may be, for example, a geometric display such as a straight line, a curve a point (including a start point and an end point) that makes a user recognize a physical difference directly derived from a chemical difference of a component of a molecule.

[Application to Manufacturing Process of Pharmaceuticals]

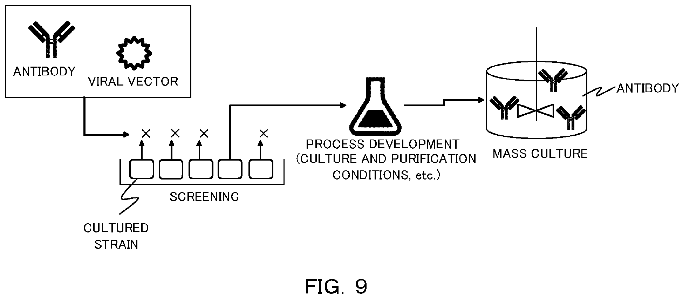

The method for analyzing the morphology of a subject molecule as described above is particularly suitable for application to a manufacturing process of a biopharmaceutical containing a protein as a component. FIG. 9 is a schematic diagram showing a manufacturing process of a biopharmaceutical. In the biopharmaceutical, cultured antibody molecules and vectors are screened under a predetermined condition, and the obtained cultured strain is selected and purified to culture a large amount of a desired biopharmaceutical.

The determinants of the conditions for culturing and purifying sources include structural identity (LC, LC/MS), sugar chain structure for a characteristic analysis, heterogeneity (activity and correlation), and completion of vector (viral capsid stability), and solutions with quality less than a certain level are excluded. In this way, the concept of "Quality-by-Design (QbD)" in which the manufacturing process is constantly monitored to optimize the manufacturing process is essential.

The method for analyzing the morphology of the subject molecule described above is effective in determining whether or not a certain quality is satisfied. It is possible to quickly and easily perform the characteristic analysis, which requires enormous time and effort for the advance method development in the conventional method of structural analysis, even for the user who does not have sufficient knowledge. Then, it is possible to reduce the cost and human burden of process optimization in translational research and quality control.

In the above embodiment, the morphological analysis method is mainly applied to an antibody molecule but may be applied to a molecule that stores and transports a substance. The ferritin molecule has a cavity therein, and the hatch part opens and closes, so that a substance to be transported can be stored therein. For example, by the above-described morphological analysis method, the opening/closing property of the hatch part of the capsid molecule can be evaluated, and the perfectness of the shape can be confirmed.

[Details of Structural Specifying Unit]

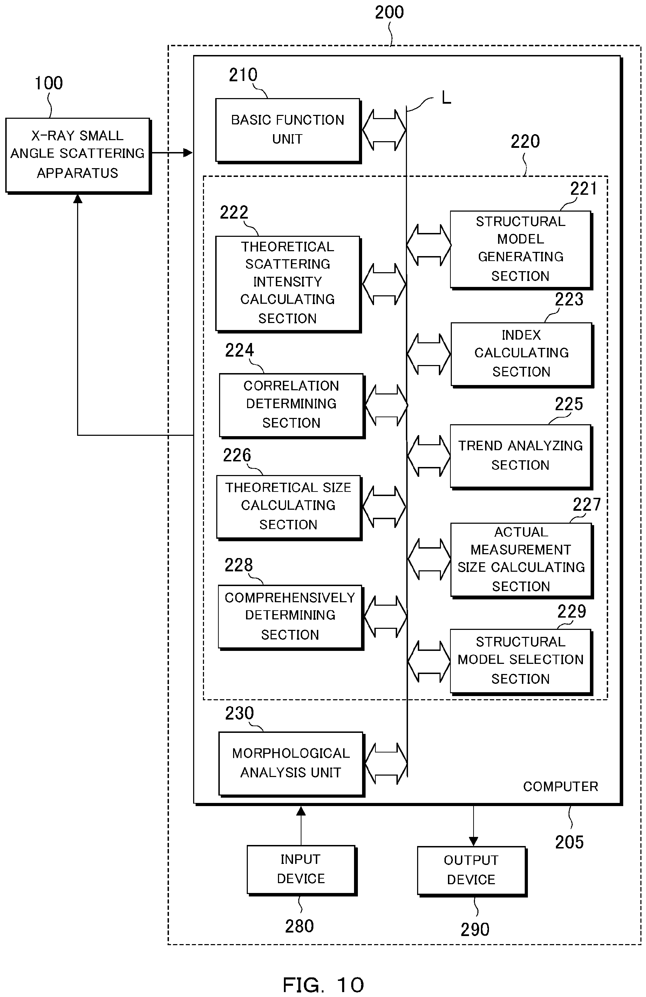

FIG. 10 is a block diagram showing details of a structure specifying unit. The morphological analysis apparatus 200 acquires data of the X-ray scattering profile measured by the small angle X-ray scattering apparatus 100 and specifies the structure of the macromolecule based on the data.

The structure specifying unit 220 comprises a structure model generating section 221, a theoretical scattering intensity calculating section 222, an index calculating section 223, a correlation determining section 224, a trend analyzing section 225, a theoretical size calculating section 226, an actual measurement size calculating section 227, a comprehensively determining section 228 and a structure model selecting section 229.

The structure model generating section 221 generates a plurality of structure models from the measured X-ray scattering profile obtained by measuring the sample S0. Generation of the structural model are described below in detail.

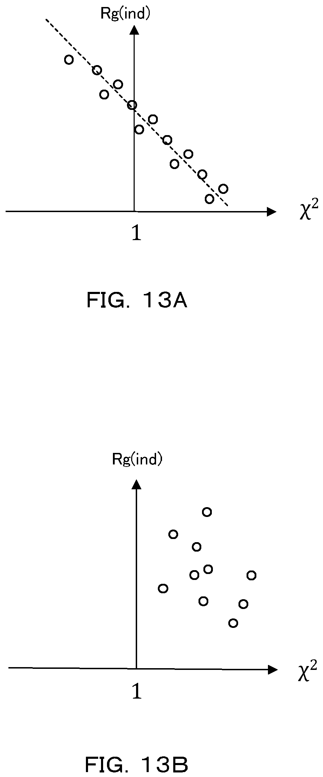

From the obtained structural model, an index $\chi^2$ indicating the degree of coincidence between the measurement data and the calculated scattering curve for each structural model and a calculation value Rg(ind) as the dynamical radius of gyration of the electron-density model can be calculated and plotted to generate a first plot (see FIG. 13 described below). The dynamic radius of gyration Rg of the molecule is a preferable example of a parameter representing the molecular size and may be another parameter representing the molecular size.

The structural model generating section 221 preferably generates a plurality of structural models by changing the condition when the distribution of the first plot tends to be. Thus, it is possible to try to regenerate the structural model when there is no validity in any of the structural models. The structural model generating section 221 may generate a plurality of structural models under a condition based on an instruction from the user. Thus, it is possible to try to generate a structural model by changing the condition when there is no validity in any of the structural models.

The structural model generating section 221 can generate each of the plurality of structural models one by one in a repetitive process according to the setting. Thus, it is possible to proceed with the processing by allocating necessary computational resources while reducing unnecessary processing and confirming the validity of the structural model. Further, the structural model generating section 221 may generate each of the plurality of structural models at one time in parallel processing according to the setting. Thus, the structural model of the biomacromolecule with high validity can be reproduced in a short time. The theoretical scattering intensity calculating section 222 calculates the theoretical scattering intensity from each of the plurality of structural models.

The index calculating section 223 calculates an index indicating the degree of coincidence between the X-ray scattering profile calculated from each of the plurality of structural models and the measured X-ray scattering profile. Specifically, $\chi^2$ is preferable as an index for which the statistical process has been performed, but the index is not particularly limited as long as it is an index indicating the degree of coincidence between the X-ray scattering profiles. In addition to $\chi^2$, parameters of a normal distribution or a Poisson distribution, R value, RMS value, RMD value and the like of indices representing the degree of coincidence between structural factors obtained from the measured diffraction data and structural factors based on the model structure obtained by the analysis are exemplified. $\chi^2$ can be calculated as follows.

$$\chi 2 = \left[ \frac{I_{exp}^{(i)}(q_j) - I_{calc}^{(i)}(q_j)}{\sigma(q_j)} \right]^2$$

$$\cdots (3)$$

scatter intensity $I_{calc}(q)$ and measured value $I_{exp}(q)$
$\sigma\,(q)$ is the standard deviation of $I_{exp}(q)$

The correlation determining section 224 generates a first plot by plotting, for each of the plurality of structural models, a parameter representing the molecular size with respect to the calculated index and determines the presence or absence of correlation of the first plot. The correlation determining section 224 preferably shows the first plot on a display. Thus, the user can visually confirm the presence or absence of correlation in the first plot.

In the case where the repetitive processing is performed, the correlation determining section 224 preferably determines whether or not there is a correlation in the plot for each round of the repetitive processing. Thus, the processing can be proceeded while confirming the validity of the structural model for each round of the repetitive processing. As a result, the processing can be efficiently performed when the computational resources are limited.

The trend analyzing section 225 performs multivariate analysis on the distribution of the first plot when there is no correlation in the first plot. Examples of the multivariate analysis include PCA (principal component analysis). Thus, for example, it is possible to determine the presence or absence of any trend even if there is no correlation. For example, even when there is no correlation in the whole data, it is possible to use various types of data as correlated data by separating a plurality of types of correlated data.

The theoretical size calculating section 226 generates a second plot by plotting, for each voxel size, a parameter representing the molecular size calculated based on the plurality of structural models. The parameter representing the calculated molecular size is preferably an intercept Rg(sect) at $\chi^2=1$ on the regression line for the calculation value Rg(ind) of the radius of gyration. Then, the theoretical size calculating section 226 calculates Rg(calc) as a calculation value of a parameter representing the molecular size of the macromolecule in the solution regardless of the voxel size using the secondary plot.

The actual measurement size calculating section 227 calculates a parameter representing the molecular size of the macromolecule in the solution from the measured X-ray scattering profile as an actual measurement value. Specifically, a Guinier plot can be performed to calculate the Rg(exp) as the actual measurement value of the dynamic radius of gyration of the molecule. When the sample forms a hydration sphere in the solution, it is preferable to treat a value obtained by subtracting a predetermined value for the hydration sphere from a value obtained from the Guinier plot as an actual measurement value Rg(exp).

The comprehensively determining section 228 determines whether or not there is statistically significant difference between the calculation value Rg(calc) of the radius of gyration calculated by the theoretical size calculating section 226 and the actual measurement value Rg(exp) calculated by the actual measurement size calculating section 227. When it is determined that there is no significant difference, a representative structural model is selected from a plurality of structural models. If it is determined that there is a significant difference, the process is terminated.

The structural model selecting section 229 selects a representative structural model from a plurality of structural models based on the calculated index. Specifically, a structural model having a $\chi^2$ close to 1 is selected as the representative structural model. By selecting a representative structural model based on the index, it is possible to accurately reproduce a structural model of a molecule in a solution having a dynamically fluctuating structure. As a result, a biomacromolecule in a solution without any advance information can be accurately visualized.

It is preferable that the structural model selecting section 229 does not select a representative structural model when there is no correlation of the first plot. Thus, when none of the structural models is valid, specification of the structural model can be stopped, and unnecessary calculation can be omitted. Then, according to the situation, the structural model can be generated again, or an experiment is conducted again for the electron density map.

The structural model selecting section 229 preferably does not select a representative structural model unless the difference between the calculation value and the measurement value is within a predetermined range. Thus, even when the structural model can be specified, the specification of the structural model can be stopped when the validity of the structural model is not guaranteed in terms of the molecular size.

[Structure Specifying Method]

(Entire Method)

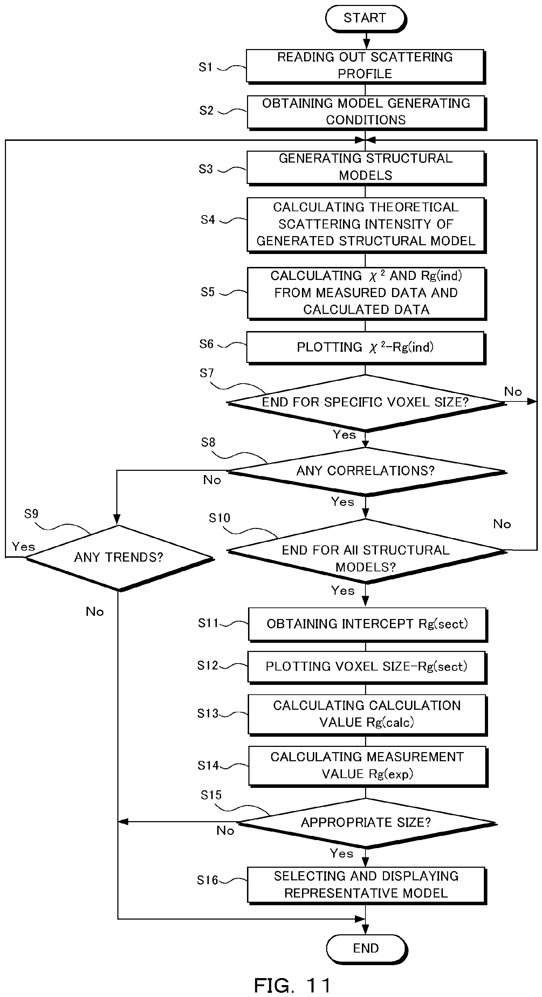

A method of specifying a structure of a macromolecule in a solution using the morphological analysis system 10 configured as described above is described. FIG. 11 is a flowchart showing the structure specifying method. First, the X-ray small-angle scattering apparatus 100 sends the sample S0 in the solution to a predetermined position and irradiates the sample S0 with X-rays. The small-angle X-ray scattering apparatus 100 detects the scattered X-rays and transmits the scattered X-rays to the computer 205 as data of a small-angle X-ray scattering profile. The computer 205 stores data of the received X-ray scattering profile.

The computer 205 receives designation by the user and reads out the X-ray scattering profile data obtained from the sample S0 to be structurally specified (step S1). Then, the generation conditions of the structural model, which are designated by the user, such as the box size, the voxel size, and the number of trials for each voxel size are obtained (step S2).

A structural model obtained from the read-out X-ray scattering profile is generated in accordance with the obtained generation conditions (step S3). The generation of the structural model are described below in detail. Next, the theoretical scattering intensity is calculated based on the generated structural model (step S4). Based on the read out measured X-ray scattering profile and the calculated theoretical scattering intensity, an index $\chi^2$ representing the degree of coincidence between the calculated X-ray scattering profile and the measured X-ray scattering profile, and a calculation value Rg(ind) for each structural model of the radius of gyration of the particle are calculated (step S5), and a plot of $\chi^2$-Rg(ind) is generated (step S6).

Next, it is determined for the repetitive condition whether or not the generation of the structural model has been tried a predetermined number of times for a particular voxel size (step S7). If it is determined that the trial has not been performed the predetermined number of times, the process returns to step S3. If it is determined that the predetermined number of trials has been performed, the process proceeds to step S8. The predetermined number of times is, for example, 50. In the above-described embodiment, the repetitive processing is performed for a particular voxel size in the step S7, but the repetitive processing may be simply performed a predetermined number of times.

In this way, it is determined whether or not the plotted $\chi^2$-Rg(ind) generated for a particular voxel size is correlated (step S8). The correlation determining process are described below in detail. When it is determined that there is no correlation, it is determined whether or not there is any trend in the plot by multivariate analysis or the like (step S9). If it is determined that there is a trend, the process returns to step S3, and the structural model is generated again with changing the condition. When it is determined that there is no trend, the series of processes is ended without selecting a representative of the structural models. When the repetitive processing of the step S7 is completed, the presence or absence of correlations is determined, and the processing with no prospect is terminated, whereby the computational resource can be efficiently used.

On the other hand, if it is determined that there is a correlation in the step S8, it is determined whether or not a condition for end of the repetition that the generation of the structural model is completed for all of the plurality of voxel sizes is satisfied (step S10), and if it is determined that the condition is not satisfied, the voxel size is changed and the process returns to the step S3.

In the step S10, if the condition of the repetition is satisfied, a regression line is obtained from the plotting of $\chi^2$-Rg(ind) according to the aggregation of structural models, and the intercept Rg(sect) at $\chi^2$=1 of the regression line is calculated (step S11). Then, a plot of the intercept Rg(sect) with respect to the voxel size is generated (step S12), a regression line is obtained based on the plot, and a calculation value Rg(calc) of the radius of gyration of the molecule is calculated as an extrapolation value where the voxel size is zero in the regression line (step S13). On the other hand, a Guinier plot is generated for the read out X-ray scattering profile, and an actual measurement value Rg(exp) of the radius of gyration of the molecules is calculated (step S14).

Next, it is determined whether or not the calculation value Rg(calc) is valid by determining whether or not the difference between the calculation value Rg(calc) of the radius of gyration and the actual measurement value Rg(exp) is within a certain range (step S15). At this time, when the sample forms a hydration sphere in the solution, it is preferable to treat a numerical value obtained by subtracting a predetermined value for the hydration sphere from a numerical value obtained from the Guinier plot as the actual measurement value Rg(exp). In the case that the hydration sphere is formed, the predetermined value is preferably 1.5 Å to 2.0 Å.

In the step S15, if the calculation value Rg(calc) is not determined to be valid, the series of processes is terminated without selecting a representative of the structural models. If it is determined that the calculation value Rg(calc) is valid, a representative structural model is selected from those having a $\chi^2$ close to 1, and the selected structural model is output to the output device 290 (step S16), and the series of processes is ended.

The representative structural model is not necessarily single and may be plural. For example, if there is a correlation for each voxel size in a plot of $\chi^2$-Rg(ind), then there may be representative structural models according to the number of correlations as an ensemble. In the above processing, representative structural models may be selected according to the number of correlations.

In the above example, each of the plurality of structural models is generated for each repetition but may be generated at once by parallel processes. Further, a certain number of parallel processes may be repeatedly performed. Which process to be chosen can be determined based on which of the computational resources and the speed of outcome is important.

(Generation of Structural Model)

Next, the generation of the structural model is described here in detail. When the biomacromolecule in the solution is irradiated with X-rays, a gradual ring-shaped scattered X-ray intensity peak is generated in the region of $q \leqq 0.7$ Å$^{-1}$. By integrating it in the circumferential direction, an X-ray small angle scattering profile is obtained. In particular, it is possible to directly visualize the electron-density that is significant to the actual structure by acquiring and analyzing the scattering intensity data in the q range up to 0.7 Å$^{-1}$ with high accuracy.

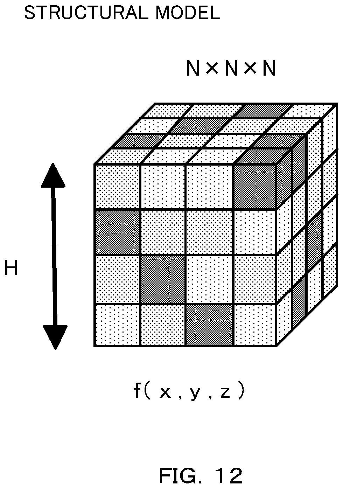

FIG. 12 is a schematic diagram showing a structural model. In the generation of the structural model, first, the volume of the cube box with one side H including the macromolecule in the real space is discretized into an $N \times N \times N$ grid of the voxels of the cube (N=4 in the example shown in FIG. 12). As indicated by the grayscale of each voxel in FIG. 12, the density f(x,y,z) of each voxel is randomly given a numerical value in a certain range. Then, the three-dimensional reciprocal lattice space intensities are calculated from the three-dimensional structural factors, and are divided into concentric shells as a function of the magnitude of the scattering vector q.

The three-dimensional scattering intensities are then transformed into a one-dimensional profile and compared to experimental scattering data. Three-dimensional structural factors are scaled to match the experimental data of the concentric shells of each q, and an inverse Fourier transform is performed to generate a new electron density map in real space. The density outside the map is set to zero. New structural factors are obtained by the forward Fourier transform and this cycle is repeated until convergence. Thus, a different structural model is generated for each trial, and the index $\chi^2$ and

the calculation value Rg(ind) are calculated for each structural model.

(Correlation Determination Process)

By plotting the $\chi^2$ and Rg(ind) calculated for each structural model and determining whether they are correlated with each other, it is possible to determine the validity of the structural model with respect to the actual measurement data. Whether or not there is a correlation in the plot can be objectively determined, for example, by using a correlation coefficient. Furthermore, when there is no correlation, by determining whether or not there is a trend in the plot, it is possible to determine whether the data is unlikely to be significant or can be significant depending on the measurement conditions.

FIGS.13A and 13B are graphs of idealized distribution and $\chi^2$-Rg(ind) distribution that cannot be analyzed, respectively. In the ideal distribution shown in FIG. 13A, $\chi^2$-Rg(ind) plots show correlations, indicating that a plurality of structural models are valid. On the other hand, in the distributions which cannot be analyzed shown in FIG. 13B, any correlations and trends do not appear in the plots of $\chi^2$-Rg(ind), and it can be seen that a plurality of structural models are not appropriate. When the structural models are appropriate, structural models having a $\chi^2$ of 1 to a predetermined range can be selected as representative structural models.

[Example 1]

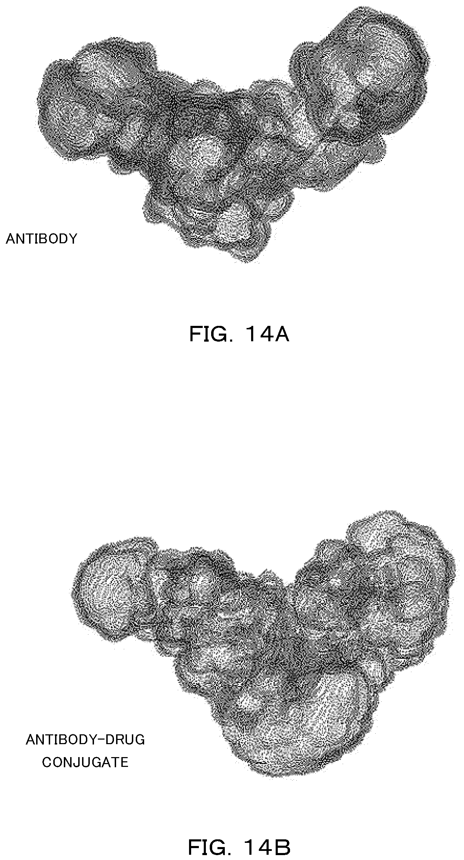

In the manufacturing process of the antibody-drug conjugate, the electron density of the source antibody molecule and the antibody-drug conjugate was observed using the above structure model specifying method. FIGS. 14A and 14B are diagrams showing electron density projections of a source antibody molecule and an antibody-drug conjugate, respectively. In both cases, the projected image of the electron density of the Y-shaped antibody molecule is shown, and it can be confirmed that the source antibody molecule with the small spread of the electron density is changed to the antibody-drug conjugate with the large spread of the electron density. The regions of the left Fab and the right Fab, the Fc of the antibody-molecule are indicated by the line 600a to 600c.

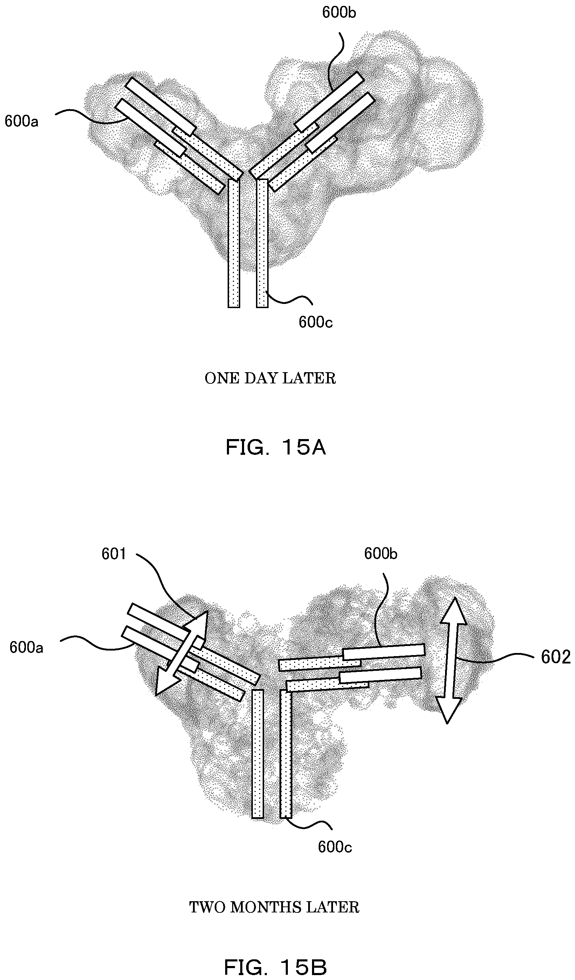

In addition, the states of the produced antibody-drug conjugate at 1 day later and 2 month later were observed using the above-described structural model specifying method. FIG. 15A and 15B show electron-density projections of the antibody molecule at day 1 and the antibody molecule at month 2, respectively, from production. As indicated by the arrows 601 and 602, it can be seen that the movement of the Fab is higher in the antibody molecule at the second month than in the antibody molecule at the first day. Over time, Fabs, which are originally formed in a Y-shape, droops, and the antibody-molecule changes from a Y-shape to a substantially open Y-shape. Further, it can be seen that the fluctuation of the Fab became large. This is considered to be the result of the deterioration progresses of the antibody-drug conjugate after the manufacture.

[Example 2]

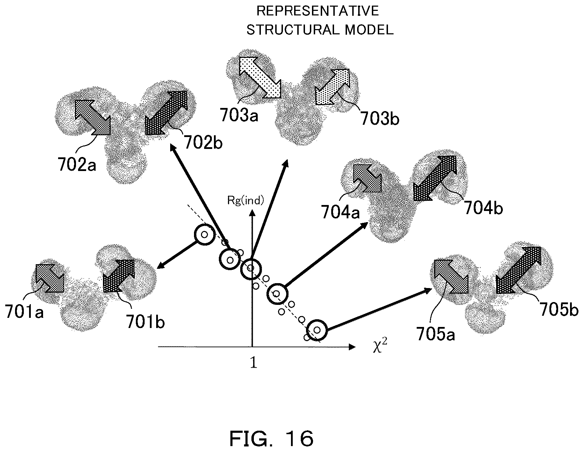

Electron-density data which can be subject data was calculated for the Y-shaped antibody molecule. The parameters were calculated by weighted average based on the observation values weighted according to the values of $\chi^2$ for the 50 structural models. By evaluating each structural model with a predetermined weight function for all the assumed structural models, objective evaluation without arbitrariness becomes possible. FIG. 16 is a schematic diagram showing structural models and points on Rg(ind) - $\chi^2$ graph. The left Fab length 701a to 705a and the right Fab length 701b to 705b of the Y-shaped antibody molecule represent the uncorrected length of the Fabs in the respective structural models. A structural model having $\chi^2$ closest to 1 is a representative structural model.

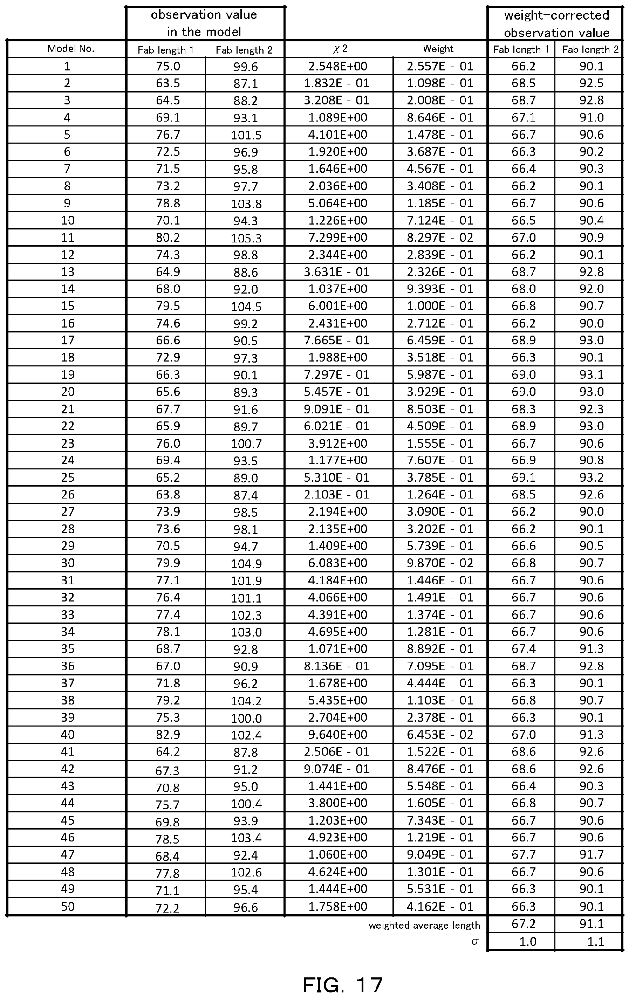

In the example shown in FIG. 16, a plurality of structural models is evaluated by weighting in accordance with $\chi^2$ determined with the respective structural models. Formula (1), in which a=0,b=4,n=10 is set, is used as the weight function. FIG. 17 is a table showing a calculation example when the left and right Fab lengths of the Y-shaped antibody molecule are weighted according to $\chi^2$. For the structural models of No.1 to 50, weight-correction observation values of the left and right Fab lengths were calculated according to $\chi^2$. In addition, the weighted average length and the standard deviation $\sigma$ were calculated by the sum of the calculated correction observation values. In this way, it is possible to calculate the parameters for the molecular shape data of the subject molecule to be analyzed. In this example, the Fab length is used as a parameter, but another parameter such as the opening angle between the arms may be used.

Description of Symbols

    10 morphological analysis system
    100 X-ray small angle scattering apparatus

110 X-ray generating section
111 X-ray source
115 optical system
117 Kratzky block
120 sample loading mechanism
125 sample holding tube
130 detector
140 control unit
200 morphological analysis apparatus
205 computer
210 basic function unit
211 I/O controlling section
215 measurement controlling section
217 measurement data storing section
220 structure specifying unit
221 structural model generating section
222 theoretical scattering intensity calculating section
223 index calculating section
224 correlation determining section
225 trend analyzing section
226 theoretical size calculating section
227 actual measurement size calculating section
228 comprehensively determining section
229 structural model selecting section
230 morphological analysis unit
234 reference data storing section
235 subject data storing section
236 output information generating section
237 morphological estimation section
238 quality determining section
280 input device
290 output device
401 to 406 human serum antibody molecule
500a to 500c, 600a to 600c mark (line segment)
501 to 503, 601, 602 mark (arrow)
504, 505 mark (enclosing line)
L control bus

## Claims

1. A morphological analysis apparatus for analyzing a morphology of molecules in a solution, comprising:

   a reference data storing section for storing reference data which is molecular shape data of a subject molecule specified in advance,
   a subject data storing section for storing subject data which is molecular shape data of the subject molecule to be analyzed, and
   a morphological estimation section for estimating a morphology of a component of a molecule in the subject data by specifying a state of a physical quantity constituting a molecular shape of the subject data with respect to the reference data, wherein
   the reference data and the subject data each have a resolution of a molecular level.

2. The morphological analysis apparatus according to claim 1, wherein
   the morphology of the component is a physical difference directly derived from a chemical difference between a component of a molecule identified by the subject data and a component of a molecule specified by the reference data.

3. The morphological analysis apparatus according to claim 1, wherein
   the morphology of the component is a difference derived from conformation and/or movement of a molecule specified

16

by the subject data with respect to a molecule specified by the reference data.

4. The morphological analysis apparatus according to claim 2 or 3, wherein
the molecule specified by the subject data is originally different from the molecule specified by the reference data.

5. The morphological analysis apparatus according to claim 2 or 3, wherein
the molecule specified by the subject data is the same molecule at different point of time with respect to the molecule specified by the reference data.

6. The morphological analysis apparatus according to any one of claims 1 to 5, wherein
the morphological estimation section specifies a state of a physical quantity constituting the molecular shape by how high or low is the physical quantity constituting the molecular shape or a shape or size of a spread represented as an aggregate of the physical quantity.

7. The morphological analysis apparatus according to any one of claims 1 to 5, wherein
the morphological estimation section uses, as the reference data, data of a physical quantity constituting a molecular shape obtained by measuring a standard sample having low movement.

8. The morphological analysis apparatus according to any one of claims 1 to 5, wherein
the morphological estimation section uses the accumulated analysis data as the reference data for the training data.

9. The morphological analysis apparatus according to any one of claims 1 to 5, further comprising an output information generating section for generating output information so as to be able to recognize a morphology of the subject data, wherein

the output information generating section causes an output device to display the subject data and receives an input of designation information that designates a component to which the morphology is to be estimated with respect to the displayed subject data, and
the morphological estimation section estimates the morphology of the designated component based on the designation information.

10. The morphological analysis apparatus according to any one of claims 1 to 5, further comprising an output information generating section for generating output information so as to recognize the morphology of the subject data, wherein
the output information generating section causes an output device to display the subject data and further makes a particular mark indicating the estimated morphology to be displayed on the subject data.

11. The morphological analysis apparatus according to any one of claims 1 to 5, wherein
the subject molecule is a Y-antibody molecule, and the component is a Fab or Fc.

12. The morphological analysis apparatus according to claim 11, further comprising:

an output information generating section for generating output information so as to be able to recognize the morphology of the subject data, and a quality determining section for determining whether or not the subject molecule conforms to a quality standard based on the estimated morphology, wherein
the output information generating section displays a non-conformance of the subject molecule with the quality standard when the subject molecule does not conform to the quality standard.

13. A morphological analysis method for analyzing a morphology of a molecule in a solution, comprising the steps of:

reading out reference data which is molecular shape data of a subject molecule specified in advance,
reading out subject data which is molecular shape data of the subject molecule to be analyzed, and
estimating a morphology of a component of a molecule in the subject data by specifying a state of a physical quantity constituting a molecular shape of the subject data with respect to the reference data, wherein
the reference data and the subject data each have a resolution of a molecular level.

14. A morphological analysis program for analyzing a morphology of a molecule in a solution, causing a computer to execute the process of:

**EP 4 542 209 A1**

reading out reference data which is molecular shape data of a subject molecule specified in advance, reading out subject data which is molecular shape data of the subject molecule to be analyzed, and estimating a morphology of a component of a molecule in the subject data by specifying a state of a physical quantity constituting a molecular shape of the subject data with respect to the reference data, wherein the reference data and the subject data each have a resolution of a molecular level.

FIG. 1

FIG. 2

FIG. 3

START

S1 — READING OUT REFERENCE DATA AND COMPONENT SPECIFICATION INFORMATION

S2 — READING OUT SUBJECT DATA

S3 — DISPLAYING SUBJECT DATA

S4 — RECEIVING DESIGNATED INPUT

S5 — ANY DESIGNATIONS?

No

Yes

S7 — SPECIFYING DESIGNATED COMPONENT

S6 — SPECIFYING All COMPONENTS

S8 — ESTIMATING MORPHOLOGY OF SPECIFIED COMPONENTS

S9 — DISPLAYING MARK

S10 — DETERMINING ESTIMATED RESULTS

S11 — DISPLAYING DETERMINATION RESULT

END

FIG. 4

401

FIG. 5A

401

500a

500b

500c

FIG. 5B

FIG. 6A

FIG. 6B

FIG. 7A

FIG. 7B

FIG. 7C

FIG. 8A

FIG. 8B

FIG. 9

200

100

FIG. 10

FIG. 11

STRUCTURAL MODEL

N × N × N

H

f( x , y , z )

FIG. 12

FIG. 13A

FIG. 13B

ANTIBODY

FIG. 14A

ANTIBODY-DRUG
CONJUGATE

FIG. 14B

600b

600a

600c

ONE DAY LATER

FIG. 15A

601

600b

600a

602

600c

TWO MONTHS LATER

FIG. 15B

REPRESENTATIVE
STRUCTURAL MODEL

703a    703b

702a    702b

704a    704b

Rg(ind)

701a    701b

705a    705b

$\chi^2$

1

FIG. 16

| Model No. | observation value in the model | | $\chi 2$ | Weight | weight-corrected observation value | |
|---|---|---|---|---|---|---|
| | Fab length 1 | Fab length 2 | | | Fab length 1 | Fab length 2 |
| 1 | 75.0 | 99.6 | 2.548E+00 | 2.557E - 01 | 66.2 | 90.1 |
| 2 | 63.5 | 87.1 | 1.832E - 01 | 1.098E - 01 | 68.5 | 92.5 |
| 3 | 64.5 | 88.2 | 3.208E - 01 | 2.008E - 01 | 68.7 | 92.8 |
| 4 | 69.1 | 93.1 | 1.089E+00 | 8.646E - 01 | 67.1 | 91.0 |
| 5 | 76.7 | 101.5 | 4.101E+00 | 1.478E - 01 | 66.7 | 90.6 |
| 6 | 72.5 | 96.9 | 1.920E+00 | 3.687E - 01 | 66.3 | 90.2 |
| 7 | 71.5 | 95.8 | 1.646E+00 | 4.567E - 01 | 66.4 | 90.3 |
| 8 | 73.2 | 97.7 | 2.036E+00 | 3.408E - 01 | 66.2 | 90.1 |
| 9 | 78.8 | 103.8 | 5.064E+00 | 1.185E - 01 | 66.7 | 90.6 |
| 10 | 70.1 | 94.3 | 1.226E+00 | 7.124E - 01 | 66.5 | 90.4 |
| 11 | 80.2 | 105.3 | 7.299E+00 | 8.297E - 02 | 67.0 | 90.9 |
| 12 | 74.3 | 98.8 | 2.344E+00 | 2.839E - 01 | 66.2 | 90.1 |
| 13 | 64.9 | 88.6 | 3.631E - 01 | 2.326E - 01 | 68.7 | 92.8 |
| 14 | 68.0 | 92.0 | 1.037E+00 | 9.393E - 01 | 68.0 | 92.0 |
| 15 | 79.5 | 104.5 | 6.001E+00 | 1.000E - 01 | 66.8 | 90.7 |
| 16 | 74.6 | 99.2 | 2.431E+00 | 2.712E - 01 | 66.2 | 90.0 |
| 17 | 66.6 | 90.5 | 7.665E - 01 | 6.459E - 01 | 68.9 | 93.0 |
| 18 | 72.9 | 97.3 | 1.988E+00 | 3.518E - 01 | 66.3 | 90.1 |
| 19 | 66.3 | 90.1 | 7.297E - 01 | 5.987E - 01 | 69.0 | 93.1 |
| 20 | 65.6 | 89.3 | 5.457E - 01 | 3.929E - 01 | 69.0 | 93.0 |
| 21 | 67.7 | 91.6 | 9.091E - 01 | 8.503E - 01 | 68.3 | 92.3 |
| 22 | 65.9 | 89.7 | 6.021E - 01 | 4.509E - 01 | 68.9 | 93.0 |
| 23 | 76.0 | 100.7 | 3.912E+00 | 1.555E - 01 | 66.7 | 90.6 |
| 24 | 69.4 | 93.5 | 1.177E+00 | 7.607E - 01 | 66.9 | 90.8 |
| 25 | 65.2 | 89.0 | 5.310E - 01 | 3.785E - 01 | 69.1 | 93.2 |
| 26 | 63.8 | 87.4 | 2.103E - 01 | 1.264E - 01 | 68.5 | 92.6 |
| 27 | 73.9 | 98.5 | 2.194E+00 | 3.090E - 01 | 66.2 | 90.0 |
| 28 | 73.6 | 98.1 | 2.135E+00 | 3.202E - 01 | 66.2 | 90.1 |
| 29 | 70.5 | 94.7 | 1.409E+00 | 5.739E - 01 | 66.6 | 90.5 |
| 30 | 79.9 | 104.9 | 6.083E+00 | 9.870E - 02 | 66.8 | 90.7 |
| 31 | 77.1 | 101.9 | 4.184E+00 | 1.446E - 01 | 66.7 | 90.6 |
| 32 | 76.4 | 101.1 | 4.066E+00 | 1.491E - 01 | 66.7 | 90.6 |
| 33 | 77.4 | 102.3 | 4.391E+00 | 1.374E - 01 | 66.7 | 90.6 |
| 34 | 78.1 | 103.0 | 4.695E+00 | 1.281E - 01 | 66.7 | 90.6 |
| 35 | 68.7 | 92.8 | 1.071E+00 | 8.892E - 01 | 67.4 | 91.3 |
| 36 | 67.0 | 90.9 | 8.136E - 01 | 7.095E - 01 | 68.7 | 92.8 |
| 37 | 71.8 | 96.2 | 1.678E+00 | 4.444E - 01 | 66.3 | 90.1 |
| 38 | 79.2 | 104.2 | 5.435E+00 | 1.103E - 01 | 66.8 | 90.7 |
| 39 | 75.3 | 100.0 | 2.704E+00 | 2.378E - 01 | 66.3 | 90.1 |
| 40 | 82.9 | 102.4 | 9.640E+00 | 6.453E - 02 | 67.0 | 91.3 |
| 41 | 64.2 | 87.8 | 2.506E - 01 | 1.522E - 01 | 68.6 | 92.6 |
| 42 | 67.3 | 91.2 | 9.074E - 01 | 8.476E - 01 | 68.6 | 92.6 |
| 43 | 70.8 | 95.0 | 1.441E+00 | 5.548E - 01 | 66.4 | 90.3 |
| 44 | 75.7 | 100.4 | 3.800E+00 | 1.605E - 01 | 66.8 | 90.7 |
| 45 | 69.8 | 93.9 | 1.203E+00 | 7.343E - 01 | 66.7 | 90.6 |
| 46 | 78.5 | 103.4 | 4.923E+00 | 1.219E - 01 | 66.7 | 90.6 |
| 47 | 68.4 | 92.4 | 1.060E+00 | 9.049E - 01 | 67.7 | 91.7 |
| 48 | 77.8 | 102.6 | 4.624E+00 | 1.301E - 01 | 66.7 | 90.6 |
| 49 | 71.1 | 95.4 | 1.444E+00 | 5.531E - 01 | 66.3 | 90.1 |
| 50 | 72.2 | 96.6 | 1.758E+00 | 4.162E - 01 | 66.3 | 90.1 |
| | | | | weighted average length | 67.2 | 91.1 |
| | | | | $\sigma$ | 1.0 | 1.1 |

FIG. 17

## INTERNATIONAL SEARCH REPORT

| | International application No. |
|---|---|
| | **PCT/JP2023/022652** |

### A. CLASSIFICATION OF SUBJECT MATTER

*G01N 23/201*(2018.01)i
FI: G01N23/201

According to International Patent Classification (IPC) or to both national classification and IPC

### B. FIELDS SEARCHED

Minimum documentation searched (classification system followed by classification symbols)

G01N23/00-23/2276, G01N33/48-33/98

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | OKUDA, Aya et al. Solution structure of multi-domain protein ER-60 studied by aggregation-free SAXS and coarse-grained MD simulation. nature. vol. 11. Springer Nature. 11 March 2021, https://www.nature.com/articles/s41598-021-85219-0<br>Structural modeling of ER-60, Aggregation-free SAXS, Coarse-grained molecular dynamics simulation, table 1, fig. 2, 4 | 1-14 |
| A | US 2020/0256811 A1 (CORNELL UNIVERSITY) 13 August 2020 (2020-08-13)<br>entire text, all drawings | 1-14 |
| A | JP 2003-177106 A (UNIV SHIMANE) 27 June 2003 (2003-06-27)<br>entire text, all drawings | 1-14 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **01 November 2023** | **21 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/022652**

| Patent document cited in search report | | | Publication date (day/month/year) | Patent family member(s) | Publication date (day/month/year) |
|---|---|---|---|---|---|
| US | 2020/0256811 | A1 | 13 August 2020 | (Family: none) | |
| JP | 2003-177106 | A | 27 June 2003 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005250721 A

**Non-patent literature cited in the description**

- **JUNICHI MATSUZAKI**. Present state and problem of the biopharmaceutical industry. *Journal of Biotechnology Society*, 2013, vol. 91 (9), 495-498